# EUROPEAN PATENT APPLICATION

(11) **EP 4 163 373 A1**
(43) Date of publication of application: **12.04.2023**
(21) Application number: 21382898.1
(22) Date of filing: 07.10.2021
(51) Int. Cl.: C12N 15/113

(54) **SPLICE SHIFTING OLIGONUCLEOTIDES FOR USE IN THE TREATMENT OF DISEASES CHARACTERIZED BY ALTERED INCLUSION OF MICROEXONS**

(71) Applicant: Universidad Autónoma de Madrid, 28049 Madrid (ES); Consejo Superior De Investigaciones Científicas, 28006 Madrid (ES); University of Southern Denmark, 5230 Odense M (DK)
(72) Inventor: RUÍZ DESVIAT, Lourdes, 28049 Madrid (ES); MARTÍNEZ PIZARRO, Ainhoa, 28049 Madrid (ES); LUCAS LOZANO, José Javier, 28006 Madrid (ES); PICÓ DEL PINO, Sara, 28006 Madrid (ES); ANDRESEN, Brage S., DK-5230 Odense M (DK)
(74) Representative: Hoffmann Eitle

(57) **Abstract**

The present invention refers to splice shifting oligonucleotides (SSOs), targeting the downstream 3' splice acceptor site relative to microexons, which are mis-spliced in various diseases, particularly in neurological diseases like autism spectrum disorders (ASD) and schizophrenia.

## Description

### FIELD OF THE INVENTION

The present invention refers to the medical field. Particularly, the present invention refers to splice shifting oligonucleotides (SSOs), targeting the downstream 3' splice acceptor site relative to microexons (hereinafter "target region"), which are mis-spliced in various diseases, particularly in neurological/neurodevelopmental diseases like autism spectrum disorders (ASD) and schizophrenia.

### STATE OF THE ART

Eukaryotic pre-mRNA molecules synthesized in the nucleus suffer several processing steps before being ready for translation in the cytoplasm. One key step in mRNA processing is splicing, by elimination of introns (or intervening sequences) which are regions of a primary transcript that are not included in the coding sequence of the mature mRNA, and resulting in the joining of exons, that comprise regulatory and coding sequences included in the mature transcript. The sites that define the exons that are spliced together (joined) are referred to as splice sites with the junction at the 5' end of the intron often called the "5' splice site, " or "splice donor site" and the junction at the 3' end of the intron called the "3' splice site" or "splice acceptor site". Correct mRNA splicing depends on conserved sequences at the intron-exon junctions, which are recognized by different factors of the spliceosome, as well as other, less well-conserved regulatory sequences anywhere in the exon or adjacent introns that either stimulate (enhancers) or repress (silencers) splicing of exons.

Alternative splicing (AS), defined as the splicing together of different combinations of exons, often results in multiple mature mRNA transcripts or transcript isoforms expressed from a single gene. Currently, there is evidence that 90% of eukaryotic genes undergo alternative splicing, thus modulating tissue specific gene expression, contributing to functional diversification. AS is a highly regulated process influenced by the splicing factors, such as SR proteins or hnRNPs, which recognize and bind to enhancers and silencers to help or hinder recruitment of the spliceosome. Recent evidence has shown that AS is widespread in the vertebrate nervous system and among the splice isoforms detected there is a subset of transcripts with inclusion, mainly in neurons, of 3-27 nucleotide long exons referred as "microexons". Their inclusion results in in the insertion of 1-9 amino acids that generally play a role in protein-protein interactions, thus defining a conserved AS program required for tissue-specific functions, more particularly in the brain, but not restricted to it. Microexon splicing depends on specific molecular mechanisms not completely characterized to date. The short length of microexons preclude the binding of splice factors involving interactions that bridge between 5' and 3' splice sites across longer exons. Evidence points to the involvement of intronic elements proximal to microexon 3' splice sites in regulation of their tissue-specific inclusion.

One major feature of the neuronal microexon splicing program is that it is frequently disrupted in the brains of autism spectrum disorder (ASD) patients. Recent analyses of post-mortem brain samples indicated that neuronal microexons displayed decreased inclusion in individuals with idiopathic autism, relative to matched controls. One prominent example characterized in detail is mis-splicing of the microexon in the CPEB4 gene (exon 4). The brains of individuals with idiopathic ASD show imbalances in CPEB4 transcript isoforms that result from decreased inclusion of this 24-nt microexon, correlating with dysregulation of mRNA polyadenylation and protein expression of ASD risk genes. Moreover, mouse models recapitulating the imbalance in CPEB4 transcript isoforms were shown to exhibit an ASD-like phenotype.

So, there is an unmet medical need of finding effective therapeutic strategies aimed at treating diseases characterized by altered inclusion of microexons. Thus, the present invention is focused on solving this problem and a strategy for finding SSOs that favour a correct microexon inclusion in those diseases in which splicing of microexons is mis-regulated, is herein provided.

### DESCRIPTION OF THE INVENTION

### Brief description of the invention

As explained above, the present invention refers to SSOs targeting the downstream 3' splice acceptor site relative to microexons which is mis-spliced in various diseases, particularly in neurological diseases like autism spectrum disorders (ASD) and schizophrenia. Specifically, the SSOs targeting the downstream 3' splice site region modulates microexon inclusion, thereby correcting the altered transcript isoform ratio associated to the disease. Therefore, this invention is directed towards treatment of diseases in which splicing of microexons is mis-regulated, particularly neurological diseases such as autism spectrum disorders (ASD) and schizophrenia.

Particularly, the inventors of the present invention have designed SSOs targeting sequences in the 3' splice acceptor site downstream of a microexon in the CPEB4 gene that exhibits altered inclusion (particularly decreased inclusion) associated to ASD and other diseases. The SSO blocks or hinders access to this region to the spliceosomal components needed for recognition and splicing of the downstream exon, slowing this process. This favours microexon recognition and inclusion in the mature mRNA. The inventors have shown that SSOs of different lengths and sequences hybridizing to this target region can induce nearly complete microexon inclusion in murine and human neuroblastoma cell lines. In terms of the splicing profile found in ASD brains, potentiation of microexon inclusion represents shifting the CPEB4 exon4-excluding (Δ4)/exon4-including (Ex4+) transcript isoform ratio towards the normal ratio found in neurotypical individuals.

So, the first embodiment of the present invention refers to SSOs specifically binding selected regions of the 3' splice acceptor site downstream of a specific microexon, for use in the treatment of diseases characterized by altered inclusion of the microexon.

In a preferred embodiment, the present invention refers to SSOs specifically binding 3' splice acceptor site downstream of microexon 4 of CPEB4 gene, for use in the treatment of neurologic diseases characterized by altered inclusion (particularly decreased inclusion) of the microexon 4 of CPEB4 gene.

In a preferred embodiment, the present invention refers to SSOs specifically binding 3' splice acceptor site downstream of microexon 4 of CPEB4 gene, for instance at a location between the nucleotides -5 and +22 relative to the first nucleotide of exon 5, for use in the treatment of neurologic diseases characterized by altered inclusion (particularly decreased inclusion) of microexon 4 of CPEB4 gene.

In a preferred embodiment, the SSO specifically binding the 3' splice acceptor site downstream of microexon 4 of CPEB4 gene is selected from the group comprising: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12 (see **Table 1**).

In a preferred embodiment, the present invention refers to the above cited SSOs for use in the treatment of neurodevelopmental/neurologic disorders such as autism spectrum disorders or schizophrenia.

The second embodiment of the present invention refers to a method for modulating the splicing of alternatively spliced microexons, or for restoring mis-spliced microexons, which comprises targeting a SSO to the 3' splice acceptor site downstream of the mis-spliced microexon.

In a preferred embodiment the present invention refers to a method for modulating the splicing of microexon 4 of CPEB4 gene, or for restoring mis-spliced microexon 4 of CPEB4 gene, which comprises targeting a SSO to the 3' splice acceptor site downstream of microexon 4 of CPEB4 gene.

In a preferred embodiment the present invention refers to a method for modulating the splicing of microexon 4 of CPEB4 gene, or for restoring mis-spliced microexon 4 of CPEB4 gene, which comprises targeting a SSO to the 3' splice acceptor site downstream of microexon 4 of CPEB4 gene in particular at a location placed between the nucleotides -5 and +22 relative to the first nucleotide of exon 5.

In a preferred embodiment, the method for modulating the splicing of microexon 4 of CPEB4 gene, or for restoring mis-spliced microexon 4 of CPEB4 gene, comprises the use of at least one of the following SSOs targeting to the 3' splice acceptor site downstream of the microexon: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12.

The third embodiment of the present invention refers to a method for screening SSOs for treatment of neurologic diseases characterized by altered inclusion of microexon 4 of CPEB4 gene, which comprises assessing whether the SSO targets the 3' splice acceptor site downstream microexon 4 of CPEB4 gene, wherein if the SSO targets the 3' splice acceptor site downstream microexon 4 of CPEB4 gene, is an indication that the SSO can be used for the treatment of neurologic diseases characterized by altered inclusion (particularly decreased inclusion) of microexon 4 of CPEB4 gene.

In a preferred embodiment, the method for screening SSOs for treatment of neurologic diseases characterized by altered inclusion (particularly decreased inclusion) of microexon 4 of CPEB4 gene, comprises assessing whether the SSO targets the 3' splice acceptor site downstream microexon 4 of CPEB4 gene, for instance at a location placed between the nucleotides -5 and +22 relative to the first nucleotide of exon 5, wherein if the SSO targets the 3' splice acceptor site downstream the microexon 4 of CPEB4 gene in particular at a location placed between the nucleotides -5 and +22 relative to the first nucleotide of exon 5, is an indication that the SSO can be used for the treatment of neurologic diseases characterized by altered inclusion (particularly decreased inclusion) of microexon 4 of CPEB4 gene.

The fourth embodiment of the present invention refers to a SSO, characterized by targeting the 3' splice acceptor site downstream microexon 4 of CPEB4 gene, selected from the group comprising: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12.

The fifth embodiment of the present invention refers to a pharmaceutical composition comprising at least one SSO selected from the group comprising: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12 and, optionally, pharmaceutically acceptable excipients or carriers.

The sixth embodiment of the present invention refers to the above-defined pharmaceutical composition for use in the treatment of neurologic diseases characterized by altered inclusion of microexon 4 of CPEB4 gene, for instance for use in the treatment of autism spectrum disorders or schizophrenia. Alternatively, this embodiment refers to a method for treating neurologic/neurodevelopmental diseases characterized by altered inclusion of microexon 4 of CPEB4 gene, for instance autism spectrum disorders or schizophrenia, which comprises administering to the patient a therapeutically effective dose or amount of the pharmaceutical composition of the invention. In the case of neural diseases, SSOs can be efficiently delivered to the central nervous system by intrathecal injection. Other delivery agents for SSOs such as exosomes or peptide conjugates have also been shown to cross the blood brain barrier [Klein AF et al., 2019. Peptide-conjugated oligonucleotides evoke long-lasting myotonic dystrophy correction in patient-derived cells and mice. J Clin Invest. 2019 Nov 1; 129(11):4739-4744. doi: 10.1172/JCI128205.PMID: 31479430*] [*Herrmann IK et al., 2021. Extracellular vesicles as a next-generation drug delivery platform. Nat Nanotechnol. 2021 Jul; 16(7) : 748-759. doi: 10.1038/s41565-021-00931-2. Epub 2021 Jul 1.PMID: 34211166 Review]*.* So, the SSOs of the invention could also be administered systemically after peptide conjugation or loaded into extracellular vesicles or exosomes.

Klein AF, Varela MA, Arandel L, Holland A, Naouar N, Arzumanov A, Seoane D, Revillod L, Bassez G, Ferry A, Jauvin D, Gourdon G, Puymirat J, Gait MJ, Furling D, Wood MJ.J Clin Invest. 2019 Nov 1;129(11):4739-4744. doi: 10.1172/JCI128205.PMID: 31479430

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included in the compositions of the invention and that causes no significant adverse toxicological effects to the patient.
- By "therapeutically effective dose or amount" of the composition of the invention is intended an amount that, when administered as described herein, brings about a positive therapeutic response in a subject having a disease in which splicing of microexons is mis-regulated. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

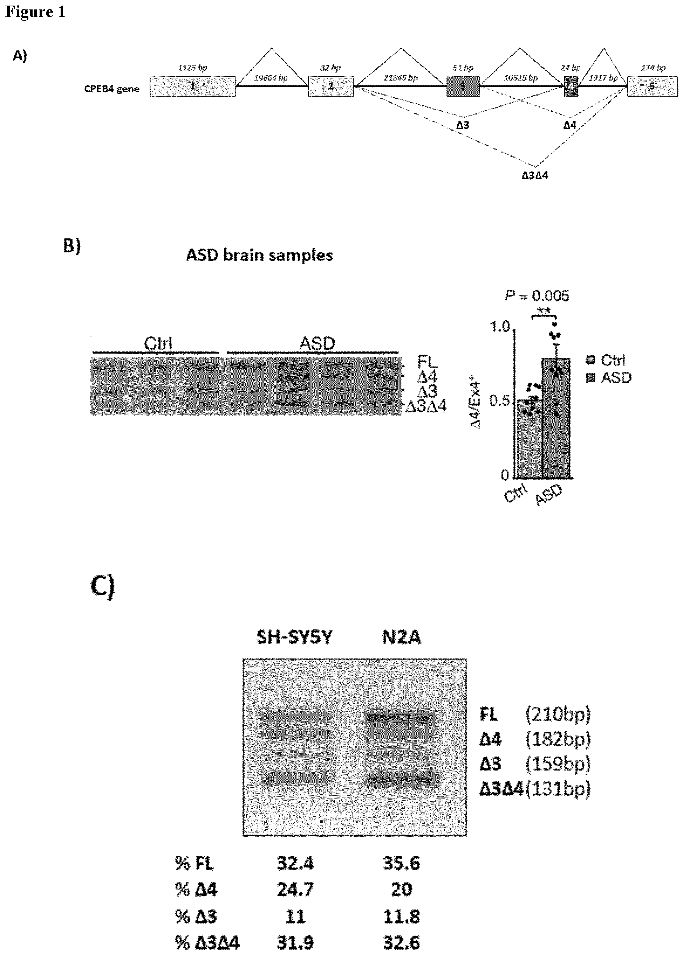

**Figure 1****. The CPEB4 gene has 4 transcript isoforms due to alternative splicing of exon 3 and microexon 4. (A)** Schematics of the CPEB4 gene indicating alternative splicing isoforms; **(B)** CPEB4 splice isoforms in control and idiopathic ASD brains and ratio of exon4-excluding (Δ4)/exon4-including (Ex4+) isoforms **(C)** CPEB4 splice isoforms in SH-SY5Y and N2A neuroblastoma cell lines. Shown on the right is the identity of the bands and their size; shown below is the estimation of the percentage of the different bands by laser densitometry. FL, full length; Δ4, without exon 4; Δ3, without exon 3; Δ3Δ4, without exons 3 and 4.

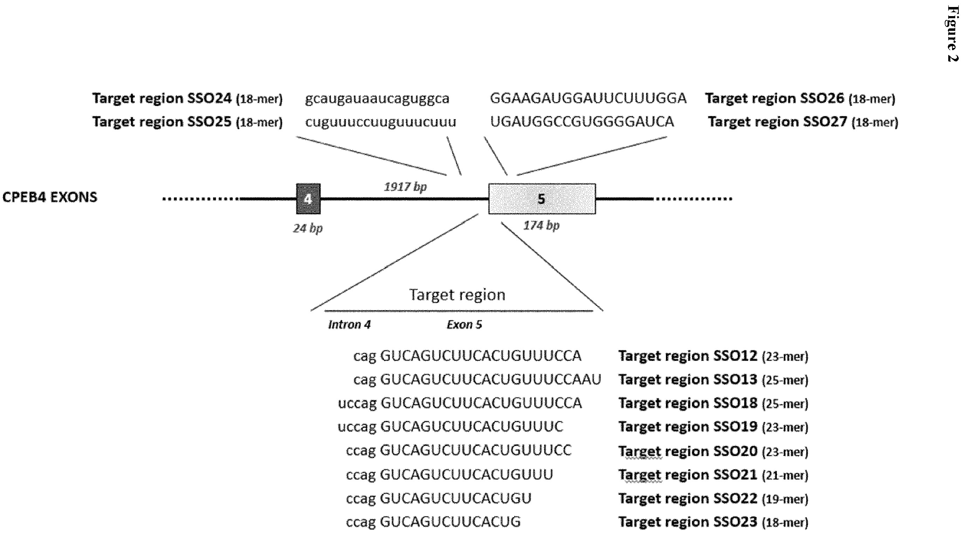

**Figure 2****. Location and target sequence of the SSOs in the 3' splice site of CPEB4 exon 5 tested to promote microexon 4 inclusion.**

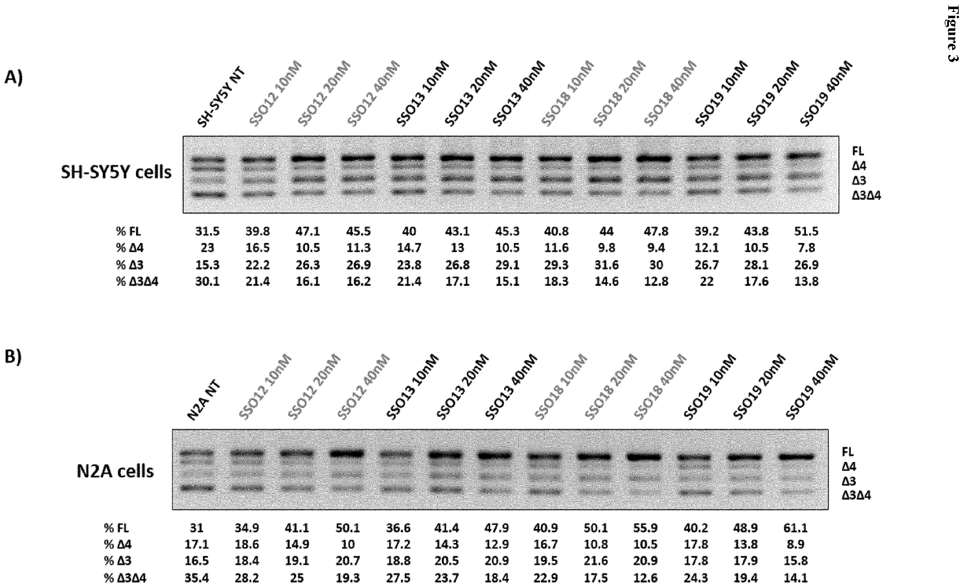

**Figure 3****. SSOs targeting the 3' splice site of CPEB4 exon 5 favour microexon 4 inclusion in neuroblastoma cells.** Gel showing the semiquantitative RT-PCR results after transfection with SSOs 12,13, 18 and 19 at different concentrations in SH-SY5Y **(A)** and N2A cells **(B)**. The identity of the bands is shown on the right and below is the estimation of the percentages of each band by laser densitometry. NT: non-transfected.

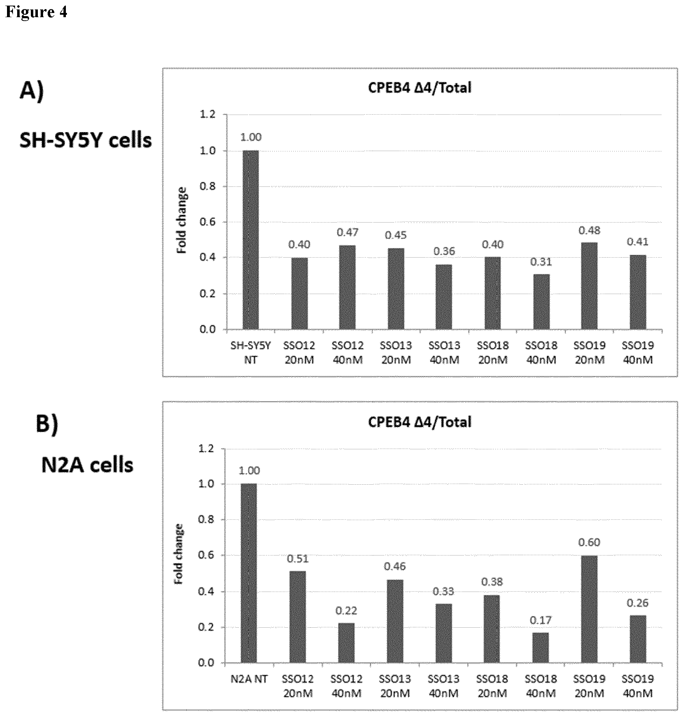

**Figure 4****. qRT-PCR analysis of CPEB4 transcript isoforms after SSO transfection.** Results obtained in SH-SY5Y **(A)** and N2A cells **(B)**, expressed as fold change in the ratio of isoforms without exon 4 and total (includes all isoforms), relative to the levels in non-transfected cells (NT).

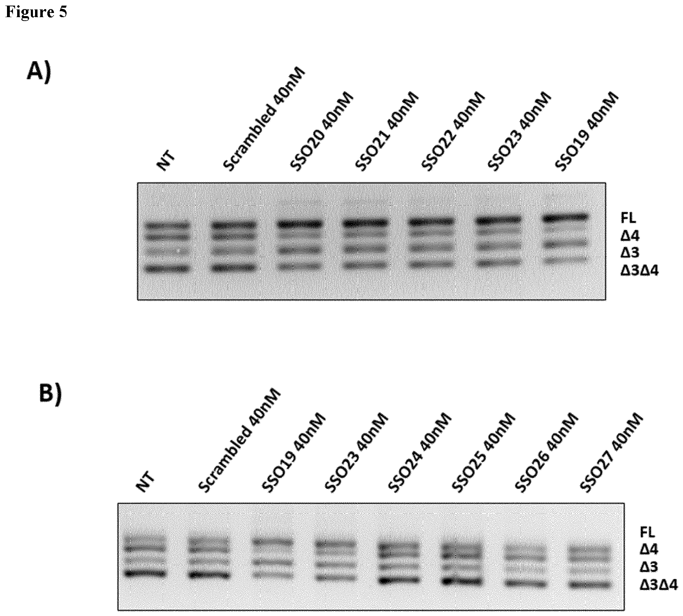

**Figure 5****. SSOs of different lengths and sequences favour microexon 5 inclusion in SH-SY5Y cells.** RT-PCR results after transfection with SSOs 20-23 **(A)** and SSOs 24-27 **(B)** using SSO19 as positive control and a scrambled oligonucleotide as negative control. The identity of the bands is shown on the right. NT: non-transfected.

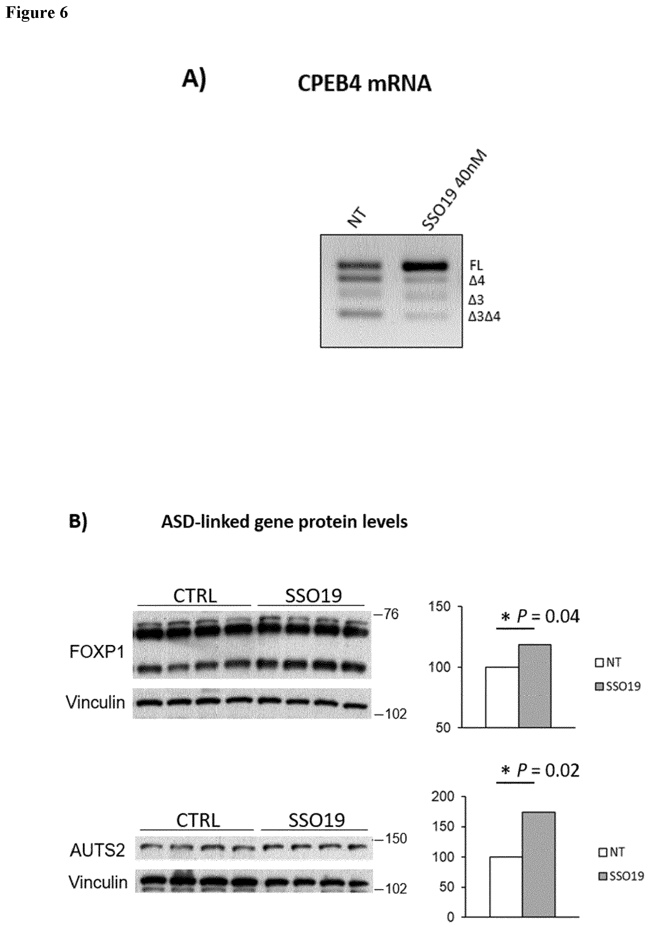

**Figure 6****. Analysis of CPEB4 target genes protein levels after SSO transfection.** CPEB4 RT-PCR analysis **(A)** and Western blot analysis of FOXP1 and AUTS2 levels **(B)** in N2A cells after transfection with SSO19. Shown on the right of each gel are the relative amounts of FOXP1 and AUTS2 levels estimated by laser densitometry. Vinculin was used as loading control. NT: non-transfected.

### Detailed description of the invention

The present invention is illustrated by means of the examples set below, without the intention of limiting its scope of protection.

### Example 1. Material and methods

### Example 1.1. SSO Design

A series of hCPEB4 (human sequence) RNA SSOs were designed and synthesized with 2'OMe modification and PS backbones, targeting the 3' splice acceptor site downstream the microexon 4 (see **Table 1**).

**Table 1**

| **CPEB4 SSO** | **Sequence 5' > 3'** | **SEQ ID NO** |
|---|---|---|
| SSO 12 | UGGAAACAGUGAAGACUGACCUG | SEQ ID NO: 1 |
| SSO 13 | AUUGGAAACAGUGAAGACUGACCUG | SEQ ID NO: 2 |
| SSO 18 | UGGAAACAGUGAAGACUGACCUGGA | SEQ ID NO: 3 |
| SSO 19 | GAAACAGUGAAGACUGACCUGGA | SEQ ID NO: 4 |
| SSO 20 | GGAAACAGUGAAGACUGACCUGG | SEQ ID NO: 5 |
| SSO 21 | AAACAGUGAAGACUGACCUGG | SEQ ID NO: 6 |
| SSO 22 | ACAGUGAAGACUGACCUGG | SEQ ID NO: 7 |
| SSO 23 | CAGUGAAGACUGACCUGG | SEQ ID NO: 8 |
| SSO 24 | UGCCACUGAUUAUCAUGC | SEQ ID NO: 9 |
| SSO 25 | AAAGAAACAAGGAAACAG | SEQ ID NO: 10 |
| SSO 26 | UCCAAGAAUCCAUCUUCC | SEQ ID NO: 11 |
| SSO 27 | UGAUCCCCACGGCCAUCA | SEQ ID NO: 12 |

A scrambled SSO (SCR: 5'-CUCAAUAUGCUACUGCCAUG-3') (SEQ ID NO: 13) not complementary to CPEB4 sequences was used as control.

### Example 1.2. Cell culture

SH-SY5Y human neuroblastoma and N2A murine neuroblastoma cells were obtained from an in-house collection. Cells were grown in Minimum Essential Medium (MEM, Sigma Aldrich) supplemented with 5% fetal bovine serum (FBS, Gibco), 1% glutamine and 0.1% antibiotic mix (penicillin/streptomycin) under standard cell culture conditions (37°C, 95% relative humidity, 5% CO₂.

### Example 1.2. Reverse transfection

Approximately 9x10⁵ cells for SH-SY5Y and 7x10⁵ for N2A were seeded in each well in a 6-well plate (Nunc) with 25 pmol (10 nM) 50 pmol (20 nM), 100 pmol (40 nM) of each 2'OMe-PS SSO, which was reverse transfected into the cells using Lipofectamine 2000 transfection reagent (Invitrogen). Forty-eight hours after transfection cells were harvested for total RNA isolation using Trizol (Invitrogen). Quantification and quality determination of RNA was done on a Nanodrop One (Thermo Scientific).

### Example 1.4. RT-PCR

cDNA synthesis was performed using 750 ng of total RNA. Splicing analysis of CPEB4 transcripts were performed by PCR with primers located in exon 2 (forward, 5'-ggacgtttgacatgcactcac-3') (SEQ ID NO: 14) and exon 5 (reverse, 5'-gaggttgatccccacggc-3') (SEQ ID NO: 15) or exon 6 (reverse, 5'-CCAAAGCGACGAAAACTAGC-3') (SEQ ID NO: 16) to amplify the four CPEB4 splicing isoforms (full-length, Δ4, Δ3, Δ3Δ4). PCR was carried out following the next protocol: 10 min at 94°C + 33 cycles (30 s at 94°C + 30 s at 58°C + 2 min at 72°C) + 10 min at 72°C.

### Example 1.5. qRT-PCR

cDNA synthesis was performed using 750 ng of total RNA. Quantification was performed by real-time PCR using a LightCycler^{®} 480 (Roche) in combination with PerfeCta SYBR^{®} Green FastMix (Quanta) and 0.25 µM of primer pair was used. The mRNA levels were normalized first relative to the GAPDH/Gapdh gene expression and then relative to total RNA in each sample.

### Example 1.6. Western blotting

Approximately 6x10⁶ N2A cells were seeded in each well in a 6-well plate (Falcon) with 100 pmol (40 nM) of each 2'OMe-PS SSOs, which was reverse transfected into the cells using Lipofectamine 2000 transfection reagent (Invitrogen). Ninety-six hours after transfection cells were frozen at -20°C and then scrapped in ice-cold extraction buffer (20 mM HEPES pH 7.4, 100 mM NaCl, 20 mM NaF, 1% Triton X-100, 1 mM sodium orthovanadate, 1 µM okadaic acid, 5 mM sodium pyrophosphate, 30 mM β-glycerophosphate, 5 mM EDTA and protease inhibitors (Complete, Roche, Cat. No 11697498001). Between 10 and 20 µg of total protein were electrophoresed on 10% SDS-polyacrylamide gels, transferred to a nitrocellulose blotting membrane (Amersham Protran 0.45 µm, GE Healthcare Life Sciences, 10600002) and blocked in TBS-T (150 mM NaCl, 20 mM Tris-HCl, pH 7.5, 0.1% Tween 20) supplemented with 5% non-fat dry milk. Membranes were incubated overnight at 4°C with the primary antibody FOXP1 (1:1000, Abcam, ab16645) and AUTS2 (1:1000, Sigma, HPA000390) in TBS-T supplemented with 5% non-fat dry milk, washed with TBS-T and next incubated with secondary HRP-conjugated anti-rabbit IgG (1:2000, DAKO, P0448) and developed using the ECL detection kit (PerkinElmer, NEL105001EA). Images were scanned with a densitometer (Bio-Rad, GS-900) and quantified with Image Lab 5.2 (Bio-Rad).

### Example 2. Results

### Example 2.1. Transcript isoforms of the CPEB4 gene in neuroblastoma cell lines

We studied the CPEB4 gene transcript isoforms present in human (SH-SY5Y) and murine (N2A) neuroblastoma cell lines, as cellular models in which to study splicing modulation by SSOs. The four transcript isoforms: full length (FL), without microexon 4 (Δ4), without exon 3 (Δ3) and without exons 3 and 4 (Δ3Δ4), were detectable in both SH-SY5Y and in N2A cells in specific ratios (**Figure 1**). The four transcript isoforms have been characterized in human brains and a recent study showed that in brains of individuals with idiopathic ASD there is an imbalance favouring isoforms lacking microexon 4 (**Figure 1**).

### Example 2.2. SSO-mediated microexon inclusion

In order to investigate how altering of splicing kinetics would affect inclusion of microexon 4, we designed overlapping SSOs of different lengths (18-mer to 25-mer) hybridizing to the region encompassing the 3' splice acceptor site downstream of microexon 4 of the CPEB4 gene and tested in SH-SY5Y and N2A cell lines (**Figure 2**). The initial results with SSOs 12, 13, 18, 19 showed that these SSOs, despite inhibiting recognition of the 3' splice acceptor site, did not result in skipping of exon 5, but instead stimulated inclusion of microexon 4 (**Figure 3**). A dose-dependent effect was observed, with the strongest effect obtained with SSO19 at 40 nM final concentration. These results were repeated by qRT-PCR analysis, confirming the decrease in the splice isoform without exon 4 (Δ4) (**Figure 4**).

Employing SSOs with different lengths we could show that stimulation of microexon 4 inclusion was dependent on the strength of SSO hybridization to the 3' splice site and even the shortest 18-mer SSO (SSO23) was effective in promoting microexon 4 inclusion, albeit at a much lower efficiency compared to 23-mer oligonucleotides (**Figure 5A**).

A series of SSOs spanning upstream and downstream in the region were designed (SSOs 24-27, see Figure 2) with the aim of defining the limits of the sequence amenable to SSO targeting for an effective microexon inclusion. The results showed that oligonucleotides covering within -5 to +22 nucleotides relative to the first nucleotide of exon 5 promoted microexon 4 inclusion. This can be defined as target region. SSOs 26-27 hybridizing downstream in exon 5 had no effect, as SSOs 24-25 targeting region upstream nucleotide -37 (**Figure 5B**). Region from -37 to -5 relative to exon 5 contain predicted branchpoint sequences and blocking them would result in undesired exon 5 skipping.

### Example 2.3. Effect on proteins regulated by CPEB4

The inventors have shown that imbalances in CPEB4 transcript isoforms that result from altered inclusion (particularly decreased inclusion) of microexon 4 is associated with a decrease in poly(A)-tail length in high-confidence ASD risk genes, correlating with reduced expression of the protein products of ASD risk genes whose transcripts are binders of CPEB4. We examined by western blotting two of these proteins, FOXP1 and AUTS2, after transfection with SSO19 in N2A and confirmed an increase in its levels (**Figure 6**

## Claims

1. Splice shifting oligonucleotides (SSOs) specifically binding selected regions of the 3' splice acceptor site downstream of a microexon, for use in the treatment of diseases **characterized by** altered inclusion of the microexon.

2. SSO, according to claim 1, specifically binding the 3' splice acceptor site downstream of microexon 4 of CPEB4 gene, for use in the treatment of neurologic diseases **characterized by** altered inclusion of microexon 4 of CPEB4 gene.

3. SSO, according to any of the previous claims, specifically binding the 3' splice acceptor site downstream of microexon 4 of CPEB4 gene at a location between nucleotides -5 and +22 relative to the first nucleotide of exon 5, for use in the treatment of neurologic diseases **characterized by** altered inclusion of microexon 4 of CPEB4 gene.

4. SSO for use, according to any of the previous claims, wherein the SSO is selected from the group comprising: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12.

5. SSO for use, according to any of the previous claims, in the treatment of neuro developmental/neurological disorders such as autism spectrum disorders or schizophrenia.

6. Method for modulating the splicing of alternatively spliced microexons, which comprises targeting a SSO to the 3' splice acceptor site downstream of the mis-spliced microexon.

7. Method, according to claim 6, for modulating the splicing of the microexon 4 of CPEB4 gene, which comprises targeting a SSO to the 3' splice acceptor site downstream of microexon 4 of CPEB4 gene.

8. Method, according to any of the claims 6 or 7, for modulating the splicing of microexon 4 of CPEB4 gene, which comprises targeting a SSO to the 3' splice acceptor site downstream of the microexon 4 of CPEB4 gene, preferentially at a location between the nucleotides -5 and +22 relative to the first nucleotide of exon 5.

9. Method, according to any of the claims 6 to 8, for modulating the splicing of microexon 4 of CPEB4 gene, which comprises the use of at least one of the following SSOs targeting to the 3'splice acceptor site downstream of the microexon: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12.

10. Method for screening SSOs for treatment of neurologic diseases **characterized by** altered inclusion of microexon 4 of CPEB4 gene, which comprises assessing whether the SSO targets the 3' splice acceptor site downstream microexon 4 of CPEB4 gene, wherein if the SSO targets the 3' splice acceptor site downstream microexon 4 of CPEB4 gene, is an indication that the SSO can be used for the treatment of neurologic diseases **characterized by** altered inclusion of microexon 4 of CPEB4 gene.

11. Method for screening SSOs for treatment of neurologic diseases **characterized by** altered inclusion of microexon 4 of CPEB4 gene, according to claim 10, which comprises assessing whether the SSO targets the 3' splice acceptor site downstream c microexon 4 of CPEB4 gene at a location between the nucleotides -5 and +22 relative to the first nucleotide of exon 5, wherein if the SSO targets the 3' splice acceptor site downstream microexon 4 of CPEB4 gene at a location placed between the nucleotides -5 and +22 relative to the first nucleotide of exon 5, is an indication that the SSO can be used for the treatment of neurologic diseases **characterized by** altered inclusion of microexon 4 of CPEB4 gene.

12. SSO **characterized by** targeting the 3' splice acceptor site downstream microexon 4 of CPEB4 gene selected from the group comprising: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12.

13. Pharmaceutical composition comprising at least one SSO according to claim 12 and, optionally, pharmaceutically acceptable excipients or carriers.

14. Pharmaceutical composition according to claim 13 for use in the treatment of neurologic diseases **characterized by** altered inclusion of microexon 4 of CPEB4 gene.

15. Pharmaceutical composition according to claim 13 or 14 for use in the treatment of neurological/neurodevelopmental diseases such as autism spectrum disorders or schizophrenia.
